(19) Europäisches Patentamt
European Patent Office
Office européen des brevets

(11) EP 0 514 791 B1

(12) EUROPÄISCHE PATENTSCHRIFT

(45) Veröffentlichungstag und Bekanntmachung des Hinweises auf die Patenterteilung:
**25.03.1998 Patentblatt 1998/13**

(51) Int. Cl.[6]: **C07D 501/00**, C07D 501/34, A61K 31/545

(21) Anmeldenummer: **92108252.5**

(22) Anmeldetag: **15.05.1992**

(54) **Kristalline Säureadditionssalze diastereomerenreiner 3-Cephem-4-carbonsäure-1-(2,2-dimethyl-propionyloxy)-ethylester**

Crystalline acid addition salts of diastereomerically pure 1-(2,2-dimethylpropionyloxy)-ethylester of 3-cephem-4-carboxylic acid

Sels cristallins d'acide d'addition d'un des deux diastéréomères pur de l'ester 1-(2,2-diméthyl-propionyloxy)-éthylique de l'acide 3-céphem-4-carboxylique

(84) Benannte Vertragsstaaten:
**AT BE CH DE DK ES FR GB GR IT LI LU NL PT SE**

(30) Priorität: **24.05.1991 DE 4116937**

(43) Veröffentlichungstag der Anmeldung:
**25.11.1992 Patentblatt 1992/48**

(73) Patentinhaber:
**HOECHST AKTIENGESELLSCHAFT**
**65926 Frankfurt am Main (DE)**

(72) Erfinder:
- **Defossa, Elisabeth, Dr.**
  **W-6270 Idstein (DE)**
- **Fischer, Gerd, Dr.**
  **W-6250 Limburg 8 (DE)**
- **Jendralla, Joachim-Heiner, Dr.**
  **W-6230 Frankfurt am Main 80 (DE)**
- **Lattrell, Rudolf, Dr.**
  **W-6240 Königstein/Ts. (DE)**
- **Wollmann, Theodor, Dr.**
  **W-6238 Hofheim am Taunus (DE)**
- **Isert, Dieter, Dr.**
  **W-6236 Eschborn (DE)**

(56) Entgegenhaltungen:
**EP-A- 0 034 536**
**EP-A- 0 049 119**
**EP-A- 0 329 008**
**EP-A- 0 402 806**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Xerox (UK) Business Services
2.15.12/3.4

## Beschreibung

Die Erfindung betrifft kristalline, enteral resorbierbare Salze der Diastereomeren des 7-[2-(2-Aminothiazol-4-yl)-2-(Z)-hydroxyimino-acetamido]-3-methoxymethyl-3-cephem-4-carbonsäure-1-(2,2-dimethylpropionyloxy)-ethylesters der Formel I

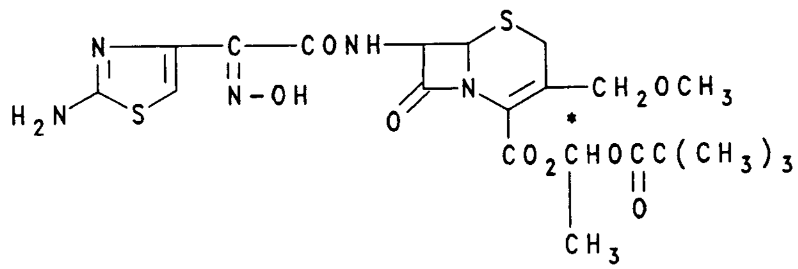

I

sowie Verfahren zu ihrer Herstellung.

In der deutschen Patentanmeldung P 38 04 841 (EP-A-0 329 008) wurden Ester von 7-[2-(2-Aminothiazol-4-yl)-2-(Z)-hydroxyimino-acetamido]-3-methoxymethyl-3-cephem-4-carbonsäure beschrieben. Von diesen ist der Ester der Formel I von besonderem Interesse, da er in verschiedenen Tierspezies gut enteral resorbiert wird und nach der Resorption durch körpereigene Enzyme wieder rasch und vollständig zum antibiotisch aktiven Cephalosporin mit freier Carboxylgruppe gespalten wird.

Anschließend wurden in der deutschen Patentanmeldung P 39 19 259 (EP-A-0 402 806) stöchiometrische, kristalline Salze aus dem Ester der Formel I und Sulfonsäuren beschrieben, die aufgrund ihrer hohen Stabilität Vorteile gegenüber der freien Base der Formel I aufweisen.

Der Ester der Formel I besitzt ein asymmetrisches Kohlenstoffatom in der 1-Stellung der Ethylestergruppe. Die in der deutschen Patentanmeldung P 39 19 259 beschriebenen Salze liegen als Mischungen der Diastereomeren vor.

Vergleichbare Mischungen von Diastereomeren liegen beim Cefotiam-Hexetil, Cefuroxim-Axetil, Cefpodoxim-Proxetil und BMY 28271 vor.

Nach den bisherigen Versuchen zum Mechanismus der enteralen Resorption derartiger Cephem-prodrugester hat die Stereochemie in der 1-Position der Ethylestergruppe keinen Einfluß auf die enterale Resorbierbarkeit. Dies konnte für die Diastereomeren von Cefotiam-Hexetil experimentell gezeigt werden (T. Nishimura et al., The Journal of Antibiotics, Vol. XL (1987) 81-90).

Daher war es sehr überraschend, daß Salze der getrennten Diastereomeren der Formel I deutliche Unterschiede bei der enteralen Resorption zeigen, so daß das besser resorbierbare Diastereomere eine höhere Bioverfügbarkeit als die in der deutschen Patentanmeldung P 39 19259 beschriebene Mischung der Diastereomeren zeigte.

Gegenstand der vorliegenden Erfindung sind daher diastereomerenreine Salze der allgemeinen Formel II, in denen das mit * gekezennzeichnete C-Atom (1S)-Konfiguration aufweist und die Gruppe = N- OH in der syn-Position steht. Das weniger polare der beiden Diastereomeren mit der (1S)-Konfiguration im Esterteil besitzt die höhere Bioverfügbarkeit.

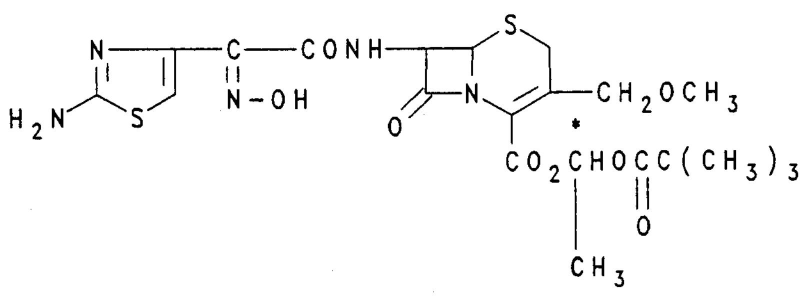

x HX II

In der allgemeinen Formel II steht HX für eine ein- oder mehrbasische Säure, wobei X ein anorganisches oder organisches, physiologisch unbedenkliches Anion sein kann.

Als anorganische Säure bedeutet HX beispielsweise stöchiometrische Menge an HCl, HBr, HJ, $HBF_4$, $HNO_3$, $HClO_4$, $H_2SO_4$ oder $H_3PO_4$. Als organische Säure steht HX für aliphatische oder aromatische Sulfonsäuren. Die anorganischen Säuren HCl, HBr und $H_2SO_4$ sowie die organischen Säuren Methansulfonsäure, Ethansulfonsäure, Benzolsulfonsäure, p-Toluolsulfonsäure und 4-Ethylbenzolsulfonsäure sind bevorzugt. Ganz besonders bevorzugt sind Benzolsulfonsäure, p-Toluolsulfonsäure und 4-Ethylbenzolsulfonsäure.

Gegenstand der Erfindung sind weiterhin Verfahren zur Herstellung von diastereomeren Verbindungen der allgemeinen Formel II, die dadurch gekennzeichnet sind, daß

1. eine Verbindung der Formel III hergestellt wird,

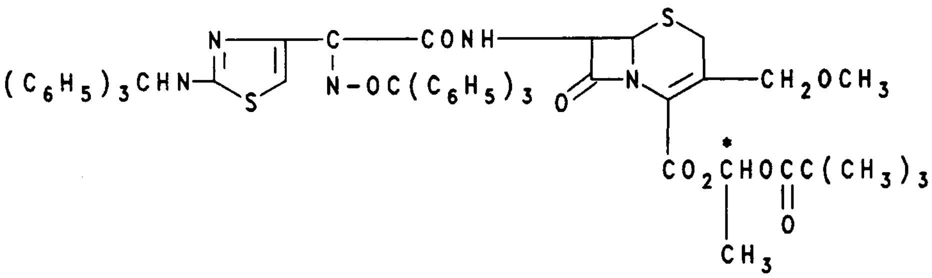

III

die Diastereomeren durch Chromatographie getrennt, die Tritylgruppen abgespalten, die Säureadditionsprodukte hergestellt werden oder

2. aus der Mischung der Diastereomeren der Formel II durch Kristallisation das weniger polare Diastereomere angereichert wird oder

3. eine Zwischenstufe der Formel IV in Form der getrennten Diastereomeren hergestellt wird

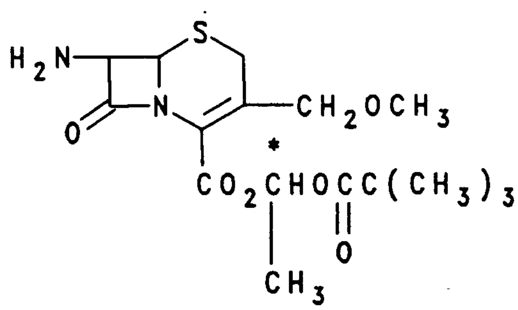

IV

und in die getrennten Diastereomeren der Formel II übergeführt wird.

Die Herstellung der bei Verfahren 1 benötigten Diastereomerenmischung der Formel III wurde bereits in der deutschen Patentanmeldung P 38 04 841 beschrieben.

Die Trennung der Diastereomeren erfolgt durch Chromatographie an Kieselgel mit einem Laufmittel aus Toluol und Ethylacetat. Das Verhältnis von Toluol zu Essigester ist breit variierbar und liegt zwischen 3 : 1 und 20: 1, wobei der Bereich von 10 : 1 bis 15 : 1 bevorzugt ist. Auf einen Teil zu trennender Mischung setzt man 20 - 80 Teile Kieselgel zur Trennung ein, wobei 30 - 50 Teile bevorzugt sind.

Die so erhaltenen reinen Diastereomeren der Formel III werden in die Salze der Formel II nach Methoden überführt, die für die Diastereomerenmischung bereits in den deutschen Patentanmeldungen P 38 04 841 und P 39 19 259 beschrieben wurden.

Nach Verfahren 2 erhält man das weniger polare Diastereomere der Formel II auch durch Kristallisation der Diastereomerenmischung aus organischen Lösungsmitteln.

Bei den üblichen Bedingungen der Umkristallisation bringt man eine Substanz in einem Lösungsmittel durch Erhitzen zum Sieden in Lösung. Verbindungen der Formeln I und II zersetzen sich unter diesen Bedingungen. Der nachfolgend beschriebene Weg ermöglicht dennoch eine Umkristallisation der Salze.

Ein Teil der Diastereomerenmischung wird zunächst in 1 - 5 Teilen, bevorzugt 1 - 2 Teilen, eines organischen Lösungsmittels wie z. B. Dimethylformamid oder Dimethylacetamid gelöst. Die so erhaltene Lösung tropft man in das 5 - 50-fache Volumen eines organischen Lösungsmittels (z. B. Alkohol, Ester, Ether, Keton, Nitril) wie z. B. Methanol, Ethanol, n-Propanol, iso-Propanol, n-Butanol, iso-Butanol, tert.-Butanol, Ethylacetat, Butylacetat, Aceton, Diethylether, Diisopropylether und Acetonitril. Besonders bevorzugt ist dabei das 10 - 20-fache Volumen an n-Propanol, iso-Propanol und n-Butanol.

Die Zutropfdauer kann zwischen 10 Minuten und 2 h liegen, bevorzugt zwischen 30 Minuten und 1 h. Zur Vervollständigung der Kristallisation rührt man noch 1 - 18 h, bevorzugt 3 - 6 h nach. Die Temperatur sollte zwischen 0 °C und 40 °C liegen, wobei 20 - 25 °C bevorzugt ist.

Die so erhaltenen Salze werden nach üblichen Laborverfahren, wie z. B. Filtration, isoliert und im Hochvakuum (< 1 Torr) in Gegenwart eines Trocknungsmittels, wie z. B. Phosphorpentoxid, von anhaftenden Lösungsmitteln befreit.

Durch mehrfaches Wiederholen des oben beschriebenen Vorganges erhält man das Diastereomer mit höherer enteraler Resorption der Formel II (HX: p-Toluolsulfonsäure) in reiner Form.

Nach Verfahren 3 stellt man die Verbindung der Formel IV, wie es in der deutschen Patentanmeldung P 38 04 841 beschrieben wurde, als Mischung der Diastereomeren her.

Die Diastereomeren können durch Kristallisation von Salzen der Formel V getrennt werden.

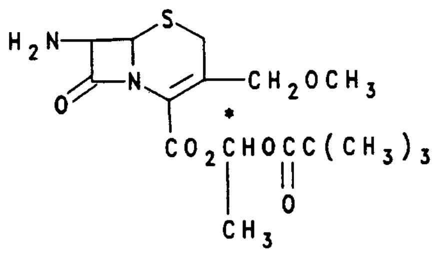 x HY V

In der allgemeinen Formel V steht HY für eine ein- oder mehrbasische Säure, wobei Y ein anorganisches oder organisches Anion sein kann.

Als anorganische Säure bedeutet HY beispielsweise HCl, HBr, HJ, HF, $HNO_3$, $HClO_4$, HSCN, $H_2SO_4$ oder $H_3PO_4$. Als organische Säure steht HY für aliphatische bzw. aromatische Sulfonsäuren, Carbonsäuren und Phosphonsäuren. So können beispielsweise die folgenden organischen Säuren eingesetzt werden: Benzolsulfonsäure, p-Toluolsulfonsäure, 4-Ethylbenzolsulfonsäure, 4-Chlorbenzolsulfonsäure, 4-Brombenzolsulfonsäure, 2-Mesitylensulfonsäure, 4-Biphenylsulfonsäure, Naphtalin-1,5-disulfonsäure, Methansulfonsäure, Ethansulfonsäure, Dodecylsulfonsäure, Camphersulfonsäure, Oxalsäure.

Als bevorzugte Säurekomponenten müssen angesehen werden: HCl, HBr, Benzolsulfonsäure, p-Toluolsulfonsäure, 4-Ethylbenzolsulfonsäure und 4-Biphenylsulfonsäure.

Die Herstellung des Salzes der Formel V erfolgt durch Zusammengeben einer Lösung der Diastereomerenmischung der Formel IV und einer Lösung der Säurekomponente HY. Als organische Lösungsmittel können z. B. Ester, Ether, Alkohole, Ketone, Kohlenwasserstoffe, Nitrile und halogenierte Kohlenwasserstoffe, sowie deren Mischungen eingesetzt werden. Bevorzugte Lösungsmittel sind z. B. Benzol, Toluol, Ethylacetat, Butylacetat, Methanol, Ethanol, n-Propanol, iso-Propanol, tert. Butanol, Diisopropylether, Acetonitril, Dichlormethan, Aceton und deren Mischungen.

Als Lösungsmittel für anorganische Säuren kann zudem Wasser eingesetzt werden, wenn das organische Lösungsmittel mit Wasser mischbar ist. Lösungen von HCl und HBr in organischen Lösungsmitteln können beispielsweise durch Einheiten von Chlorwasserstoff oder Bromwasserstoff erzeugt werden oder auch aus Acetylhalogeniden, Phosphorhalogeniden und Phosphoroxyhalogeniden und einem Alkohol (Halogen = Cl, Br).

Wichtig für eine Anreicherung eines Diastereomeren ist das Verhältnis der Base der Formel IV zur Säurekomponente. Für ein Äquivalent der Diastereomerenmischung sollten 0,2 - 2,0, bevorzugt 0,3 bis 1,0 Äquivalente Säurekomponente eingesetzt werden.

Die Zugabe der Säurekomponente erfolgt bei Raumtemperatur. Abhängig von der Säurekomponente und dem Lösungsmittel rührt man zur Vervollständigung der Fällung noch bis zu 10 Stunden nach. Gegebenenfalls muß zur Vervollständigung der Fällung auf Temperaturen zwischen Raumtemperatur und - 78 °C gekühlt werden.

Die nach Filtration erhaltenen Salze werden falls notwendig durch Kristallisation weiter gereinigt. Dazu werden die oben beschriebenen Lösungsmittel und deren Mischungen eingesetzt. Die Auswahl des optimalen Lösungsmittels hängt von der verwendeten Säurekomponente ab. So sind z. B. für das p-Toluolsulfonsäuresalz Methanol, Ethanol, n-Propanol und iso-Propanol geeignet.

Das Verfahren ist dadurch gekennzeichnet, daß die Fällung der Diastereomeren der allgemeinen Formel IV in zwei aufeinanderfolgenden Teilschritten erfolgt. So wird beispielsweise durch Zusammengeben einer Lösung des Diastereomerengemisches der Formel IV mit einer Lösung der Säurekomponente HY zunächst das schwerer lösliche Diastereomere der allgemeinen Formel V gefällt, durch Filtration abgetrennt, und anschließend aus der Filtrationslösung das leichter lösliche Diastereomere der allgemeinen Formel V gefällt. Bei den aufeinanderfolgenden Teilschritten kann die Säurekomponente HY gleich oder verschieden sein, wobei die Reihenfolge der Zugabe unterschiedlicher Säurekomponenten HY beliebig ist. So kann beispielsweise durch geeignete Wahl der Säurekomponente HY zunächst das polarere Diastereomere der allgemeinen Formel IV oder das unpolarere Diastereomere der allgemeinen Formel IV als schwerer lösliches Salz gefällt werden.

Durch die Wahl der Säurekomponente können beide Diastereomere der Formel V in reiner Form erhalten werden. So erhält man z. B. bei Verwendung von Chlorwasserstoff oder Bromwasserstoff das polarere Diastereomere, während die Verwendung von Benzolsulfonsäure, 4-Ethylbenzolsulfonsäure, Biphenyl-sulfonsäure oder p-Toluolsulfonsäure das weniger polare Diastereomere liefert.

Alternativ kann man Diastereomerenmischungen der Formel IV auch ausgehend von Verbindungen der Formel VI erhalten.

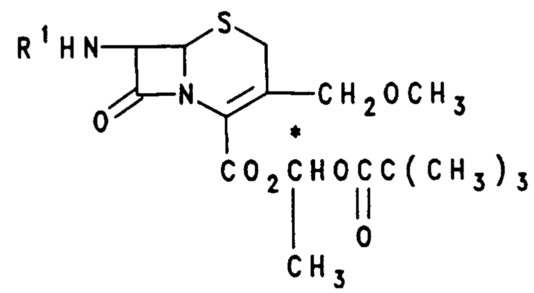

VI

Die Gruppe $R^1$ stellt dabei eine in der Peptidchemie übliche Aminoschutzgruppe, wie z. B. die Formylgruppe, die tert.-Butoxycarbonylgruppe, die Phenoxyacetylgruppe, die Phenylacetylgruppe, die Allyloxycarbonylgruppe, die Benzyloxycarbonylgruppe und die 4-Nitrobenzyloxycarbonylgruppe, dar.

Die Abspaltung der Schutzgruppen erfolgt nach an sich bekannten Methoden. So kann die Formylgruppe und die tert.-Butyloxycarbonylgruppe beispielsweise mit Säure abgespalten werden. Die Phenoxyacetylgruppe und die Phenylacetylgruppe kann beispielsweise mit Phosphorpentachlorid oder auch enzymatisch mit Penicillin-Acylasen abgespalten werden. Bei der Allyloxycarbonylgruppe kann die Abspaltung mit $Pd[P(C_6H_5)_3]_4$ erfolgen. Die Benzyloxycarbonylgruppe und die 4-Nitrobenzyloxycarbonylgruppe können hydrogenolytisch entfernt werden.

Bei der Abspaltung der Phenoxyacetylgruppe oder der Phenylacetylgruppe mit Phosphorpentachlorid erhält man bei zügiger Aufarbeitung in angereicherter Form das polarere Diastereomere als Hydrochlorid auch ohne Chlorwasserstoffzusatz. Als Quelle für den Chlorwasserstoff dienen bei der Aufarbeitung nicht entfernte Phosphorsäureesterchloride, die langsam Chlorwasserstoff freisetzen.

Ausgehend von Verbindungen der Formel VI kann man auch zu diastereomerenreinen Verbindungen der Formel V kommen, indem man zunächst die Trennung der Diastereomeren durchführt und dann die Schutzgruppe abspaltet. Die Trennung der Diastereomeren kann durch Kristallisation oder Chromatographie erfolgen, wobei die genauen Bedingungen von der Schutzgruppe $R^1$ abhängen. Steht $R^1$ z. B. für die Phenoxyacetylgruppe, so können die Diastereomeren durch Chromatographie an Kieselgel mit einem organischen Lösungsmittelgemisch getrennt werden.

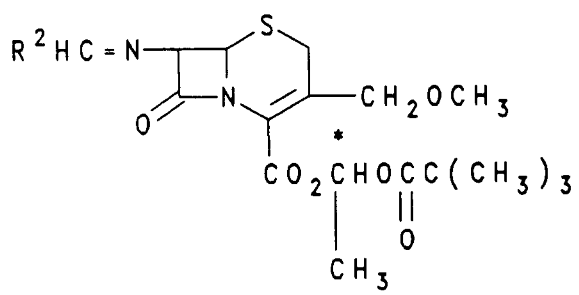

VII

Eine weitere Alternative zur Herstellung von reinen Diastereomeren der Formel IV geht von Schiffschen Basen der Formel VII aus, worin $R^2$ einen Phenyl- oder Naphthylrest darstellt, der durch $(C_1$-$C_4)$-Alkyl, Phenyl, Methoxy, Halogen (z. B. F, Br, Cl, I) oder Nitro substituiert sein kann.

Das Diastereomerengemisch der Schiffschen Basen der Formel VII wird entweder durch Chromatographie, z. B. an Kieselgel, oder durch fraktionierte Kristallisation getrennt. Die Rückspaltung der Schiffschen Basen zu den reinen

Diastereomeren der Formel IV erfolgt nach an sich bekannten Methoden, z. B. durch saure Hydrolyse oder mittels Girard-T-Reagens.

Ausgehend von den diastereomerenreinen Salzen der Formel V stellt man nach bekannten Methoden die diastereomeren Basen der Formel IV her und überführt diese, wie in den deutschen Patentanmeldungen P 38 04 841 und P 39 19 259 beschrieben, in die diastereomeren Salze der Formel II.

Die Nützlichkeit der vorliegenden Erfindung liegt in einer erhöhten enteralen Resorption für das weniger polare Diastereomer der Formel II, wie es in Tabelle 1 für das p-Toluolsulfonsäuresalz gezeigt wird.

Tabelle 1

| Diastereomerenzusammensetzung | Wiederfindungsrate (Mittelwert aus vier Versuchen) |
|---|---|
| Diastereomer 1 aus Beispiel 1 | 67,7 % |
| Diastereomer 2 aus Beispiel 1 | 19,7 % |
| Diastereomerenmischung (Verhältnis 1/1) | 39,2 % |

Tabelle 1 zeigt die Wiederfindungsrate (0 - 24 h) von 7-[2-(2-Aminothiazol-4-yl)-2-(Z)-hydroxyimino-acetamido]-3-methoxymethyl-3-cephem-4-carbonsäure im Urin von Hunden (n = 4) nach oraler Gabe von 7-[2-(2-Aminothiazol-4-yl)-2-(Z)-hydroxyimino-acetamido]-3-methoxymethyl-3-cephem-4-carbonsäure-1-(2,2-dimethyl-propionyloxy)-ethylester-p-toluolsulfonat (Dosis: 10 mg/kg bezogen auf den antibakteriell aktiven Wirkstoff). Die Wirkstoffmenge im Urin wurde mikrobiologisch durch einen Agar-Diffusionstest unter Verwendung von Mueller-Hinton-Agar (mit 10 % Schafsblut) und Streptococcus pyogenes A77 als Testkeim bestimmt.

Die erfindungsgemäßen Verbindungen der allgemeinen Formel II werden in Form von üblichen pharmazeutischen Zubereitungen, wie z. B. Kapseln, Tabletten, Pulvern, Sirupen oder Suspensionen oral verabreicht. Die Dosis hängt vom Alter, den Symptomen und dem Körpergewicht des Patienten sowie von der Dauer der Behandlung ab. Sie liegt jedoch in der Regel zwischen etwa 0,1 g und etwa 5 g täglich, vorzugsweise zwischen etwa 0,2 g und etwa 3 g täglich. Die Verbindungen werden vorzugsweise in aufgeteilten Dosen verabreicht, beispielsweise 2 bis 4mal täglich, wobei die Einzeldosis beispielsweise zwischen 50 und 500 mg Wirkstoff enthalten kann.

Die oralen Zubereitungen können die üblichen Trägerstoffe und/oder Verdünnungsmittel enthalten. So kommen beispielsweise für Kapseln oder Tabletten Bindemittel, wie z. B. Gelatine, Sorbitol, Polyvinylpyrrolidon oder Carboxymethylcellulose, Verdünnungsmittel, wie z. B. Lactose, Zucker, Stärke, Calciumphosphate oder Polyethylenglykol, Gleitstoffe, wie z. B. Talkum oder Magnesiumstearat in Betracht. Für flüssige Zubereitungen, z. B. wäßrige oder ölige Suspensionen, sind Sirupe oder ähnliche bekannte Zubereitungsformen geeignet.

Die folgenden Ausführungsbeispiele für erfindungsgemäß herstellbare, diastereomerenreine Salze der Verbindungen der Formel I, 7-[2-(2-Aminothiazol-4-yl)-2-(Z)-hydroxyimino-acetamido]-3-methoxymethyl-3-cephem-4-carbonsäure-1-(2,2-dimethylpropionyloxy)-ethylester, dienen zur weiteren Erläuterung der Erfindung. ein.

Ausführungsbeispiel 1:

Vorstufe:

2-(2-Tritylaminothiazol-4-yl)-2-(Z)-trityloximino-essigsäurechlorid

Zu einer Lösung von 42.0 g (54 mmol) 2-(2-Tritylaminothiazol-4-yl)-2-(Z)-trityloximinoessigsäure-triethylammoniumsalz in 400 ml wasserfreiem Methylenchlorid tropft man bei - 70 °C innerhalb von 30 min 11.4 g (55 mmol) Phosphorpentachlorid, gelöst in 200 ml wasserfreiem Methylenchlorid, so zu, daß die Innentemperatur - 50 °C nicht übersteigt. Nach weiteren 60 min bei - 70 °C wird das Lösungsmittel im Vakuum entfernt, wobei die Badtemperatur 30 °C nicht übersteigen sollte. Anschließend wird noch kurz im Hochvakuum getrocknet. Das so erhaltene Rohprodukt wird in 100 ml wasserfreiem Methylenchlorid gelöst und direkt zur Acylierung eingesetzt.

Stufe 1:

3-Methoxymethyl-7-[2-(2-tritylaminothiazol-4-yl)-2-(Z)-trityloximinoacetamido]-3-cephem-4-carbonsäure-1-(2,2-dimethylpropionyloxy)-ethylester

Zu einer Suspension von 14.0 g (57 mmol) 7-Amino-3-methoxymethyl-3-cephem-4-carbonsäure in 160 ml wasserfreiem Methylenchlorid gibt man bei 0 °C langsam 9.5 ml (64 mmol) DBU und rührt 30 min. bei 0 °C nach. Dann setzt man 20.8 g (81 mmol) 2,2-Dimethylpropionsäure-1-iodethylester zu, rührt noch 30 min bei 0 °C und läßt dann innerhalb von 30 min auf Raumtemperatur aufwärmen. Nach dem erneuten Abkühlen auf 0 °C tropft man das rohe 2-(2-Tritylaminothiazol-4-yl)-2-(Z)-trityloximinoessigsäurechlorid (~ 54 mmol) gelöst in 100 ml Methylenchlorid zu und rührt dann noch 2 h bei 0 °C. Die Reaktionsmischung wird im Vakuum eingeengt und der Rückstand in Ethylacetat aufgenommen. Nacheinander wird mit 5-%iger Natriumthiosulfatlösung, gesättigter Natriumhydrogencarbonatlösung und gesättigter Kochsalzlösung gewaschen, die organische Phase über Natriumsulfat getrocknet und im Vakuum zur Trockne eingeengt. Das Rohprodukt wird durch Chromatographie an Kieselgel (35 - 70µm) gereinigt (Säule: 50 cm x 8.5 cm, Toluol/Ethylacetat = 5/1).

Ausbeute: 36.5 g (66 %)

Die Diastereomeren liegen im Verhältnis 1/1 vor.

Stufe 2:

Chromatographische Trennung der Diastereomeren

17 g der Diastereomerenmischung werden an Kieselgel (35 - 70 µm, Säule: 46 x 7.5 cm) mit Toluol/Ethylacetat (15/1) und einer Flußrate von 50 ml/min. chromatographiert. Nach dem Einengen im Vakuum erhält man 6.0 g des unpolaren Diastereomeren 1 und 4.5 g des polaren Diastereomeren 2.

Diastereomer 1:

$R_f$ (Toluol/Ethylacetat = 5/1): 0.48
$^1$H-NMR (DMSO-$d_6$, 270 MHz): $\delta$ = 1.15 (s, 9H, $C(CH_3)_3$); 1.50 (d, 3H, $OCH(CH_3)O$); 3.20 (s, 3H, $OCH_3$); 3.57 (AB-System, 2H, $SCH_2$); 4.15 (s, 2H, $CH_2O$); 5.25 (d, 1H, H-6); 5.89 (dd, 1H, H-7); 6.59 (s, 1H, Thiazol-H); 6.89 (q, 1H, $OCH(CH_3)O$); 7.12 - 7.37 (m, 30H, Aromaten-H); 8.75 (s, 1H, NH); 9.90 (d, 1H, Amid-NH).

Diastereomer 2:

$R_f$ (Toluol/Ethylacetat = 5/1): 0.40
$^1$H-NMR ($CDCl_3$, 270 MHz): $\delta$ = 1.22 (s, 9H, $C(CH_3)_3$); 1.56 (d, 3H, $OCH(CH_3)O$); 3.30 (s, 3H, $OCH_3$); 3.39 (AB-System, 2H, $SCH_2$); 4.27 (s, 2H, $CH_2O$); 5.05 (d, 1H, H-6); 6.04 (dd, 1H, H-7); 6.41 (s, 1H, Thiazol-H); 6.75 (s, 1H, NH); 7.04 (q, 1H, $OCH(CH_3)O$); 7.10 - 7.44 (m, 30H, Aromaten-H).

Stufe 3:

7-[2-(2-Aminothiazol-4-yl)-2-(Z)-hydroxyiminoacetamido[-3-methoxymethyl-3-cephem-4-carbonsäure-1-(2,2-dimethylpropionyloxy)-ethylester

Diastereomer 1:

Eine Lösung von 6.0 g (5.9 mmol) des Diastereomeren 1 aus Stufe 2 in 15 ml Ameisensäure wird tropfenweise mit 3 ml Wasser versetzt. Zunächst wird 90 min bei Raumtemperatur und dann 30 min bei 0 °C gerührt. Das ausgefallene Triphenylcarbinol wird abgesaugt und mit wenig Ameisensäure/Wasser (5/1) nachgewaschen. Die vereinigten Filtrate werden mit 60 ml Ethylacetat und 20 ml Wasser versetzt. Unter Kühlung im Eisbad stellt man mit 2 N Natronlauge den pH-Wert auf 3.0 ein. Die organische Phase wird abgetrennt, zweimal mit je 50 ml Wasser gewaschen und erneut mit 50 ml Wasser versetzt. Durch Zusatz von 40%-iger Natronlauge stellt man den pH-Wert auf 6.5 ein, wobei die Innentemperatur 10°C nicht übersteigen darf. Nach dem Abtrennen wird die organische Phase mit gesättigter Kochsalzlösung gewaschen, über Natriumsulfat getrocknet und im Vakuum auf ein Viertel des Ausgangsvolumens eingeengt. Die so erhaltene Lösung wird in 150ml Diisopropylether eingetropft. Nach weiteren 60 min Rühren bei Raumtemperatur

saugt man das Produkt ab, wäscht mit Diisopropylether nach, trocknet zunächst 18 h an der Luft und dann im Vakuum über Phosphorpentoxid.

Ausbeute: 1.7 g (54 %)
$^1$H-NMR (DMSO-$d_6$, 270 MHz): $\delta$ = 1.15 (s, 9H, $C(CH_3)_3$); 1.48 (d, 3H, $OCH(CH_3)O$); 3.20 (s, 3H, $OCH_3$); 3.55 (AB-System, 2H, $SCH_2$); 4.13 (s, 2H, $CH_2O$); 5.21 (d, 1H, H-6); 5.85 (dd, 1H, H-7), 6.65 (s, 1H, Thiazol-H); 6.87 (q, 1H, $OCH(CH_3)O$); 7.11 (s, 2H, $NH_2$); 9.47 (d, 1H, Amid-NH); 11.28 (s, 1H, NOH).

Diastereomer 2:

Ebenso werden 4.5 g (44 mmol) des in Stufe 2 erhaltenen Diastereomeren 2 umgesetzt.

Ausbeute: 1.7 g (71 %)
$^1$H-NMR (DMSO-$d_6$, 270 MHz): $\delta$ = 1.16 (s, 9H, $C(CH_3)_3$); 1.49 (d, 3H, $OCH(CH_3)O$); 3.20 (s, 3H, $OCH_3$); 3.55 (AB-System, 2H, $SCH_2$); 4.12 (s, 2H, $CH_2O$); 5.19 (d, 1H, H-6); 5.82 (dd, 1H, H-7); 6.66 (s, 1H, Thiazol-H); 6.93 (q, 1H, $OCH(CH_3)O$); 7.10 (s, 2H, $NH_2$); 9.45 (d, 1H, Amid-NH); 11.29 (s, 1H, NOH).

Stufe 4:

7-[2-(2-Aminothiazol-4-yl)-2-(Z)-hydroxyiminoacetamido]-3-methoxymethyl-3-ce phem-4-carbonsäure-1-(2,2-dimethyl-propionyloxy]-ethylester-p-toluolsulfonat

Diastereomer 1:

Eine Suspension von 1 g (1.85 mmol) Diastereomer 1 aus Stufe 3 in 35 ml n-Propanol wird mit 383 mg (2.0 mmol) p-Toluolsulfonsäure-monohydratin 1 ml n-Propanol versetzt. Der Feststoff geht in Lösung und nach wenigen Minuten beginnt das Salz zu kristallisieren. Bei Raumtemperatur wird noch 1 h gerührt, dann wird das Produkt abgesaugt und mit 5 ml n-Propanol und 10 ml Diisopropylether gewaschen. Zunächst wird 18 h an der Luft und dann im Hochvakuum über Calciumchlorid und Paraffin getrocknet.

Ausbeute: 1.09 g (83 %)
$[\alpha]_D^{20}$ = + 48,8 (c=1, Methanol)
Smp. > 200°C (Zersetzung)
$^1$H-NMR (DMSO-$d_6$, 270 MHz): $\delta$ = 1.15 (s, 9H, $C(CH_3)_3$); 1.48 (d, 3H, $OCH(CH_3)O$); 2.29 (s, 3H, Aryl-$CH_3$); 3.20 (s, 3H, $OCH_3$); 3.59 (AB-System, 2H, $SCH_2$); 4.14 (s, 2H, $CH_2O$); 5.24 (d, 1H, H-6); 5.85 (dd, 1H, H-7); 6.82 (s, 1H, Thiazol-H); 6.87 (q, 1H, $OCH(CH_3)O$); 7.08-7.15 und 7.45-7.52 (2 x m, 2 x 2H, Aromaten-H); 8.0-8.8 (br., 3H, $NH_3$); 9.67 (d, 1H, Amid-NH); 12.04 (s, 1H, NOH).

Diastereomer 2:

Ausgehend von 1.6 g (2.5 mmol) Diastereomer 2 aus der Stufe 3 stellte man durch Kristallisation aus 15 ml n-Propanol das p-Toluolsulfonsäuresalz her.

Ausbeute: 1.4 g (66 %)
$[\alpha]_D^{20}$ = + 12,7 (c = 1, Methanol)
Smp. > 200°C (Zersetzung)
$^1$H-NMR (DMSO-$d_6$, 270 MHz): $\delta$ = 1.17 (s, 9H, $C(CH_3)_3$); 1.49 (d, 3H, $OCH(CH_3)O$); 2.29 (s, 3H, Aryl-$CH_3$); 3.21 (s, 3H, $OCH_3$); 3.57 (AB-System, 2H, $SCH_2$); 4.13 (s, 2H, $CH_2O$); 5.22 (d, 1H, H-6); 5.82 (dd, 1H, H-7); 6.85 (s, 1H, Thiazol-H); 6.94 (q, 1H, $OCH(CH_3)O$); 7.08-7.16 und 7.45-7.52 (2 x m, 2 x 2H, Aromaten-H); 8.4-8.9 (br., 3H, $NH_3$); 9.68 (d, 1H, Amid-NH); 12.12 (s, 1H, NOH).

Ausführungsbeispiel 2:

7-[2-(2-Aminothiazol-4-yl)-2-(Z)-hydroxyiminoacetamido]-3-methoxymethyl-3-cephem-4-carbonsäure-1-(2,2-dimethyl-propionyloxy)-ethylester-p-toluolsulfonat (Diastereomer 1)

50 g (70 mmol) 7-[2-(2-Aminothiazol-4-yl)-2-(Z)-hydroxyiminoacetamido]-3-methoxymethyl-3-cephem-4-carbonsäure-1-(2,2-dimethylpropionyloxy)-ethyles ter-p-toluolsulfonat (Diastereomer 1 / Diastereomer 2 = 63/37) werden

unter vorsichtigem Erwärmen in 65 ml Dimethylacetamid gelöst. Innerhalb 1 h tropft man die Lösung in 450 ml n-Propanol ein. Zur Vervollständigung der Kristallisation rührt man noch 4 h bei Raumtemperatur, saugt das Produkt ab, wäscht nacheinander mit n-Propanol und Diisopropylether und trocknet zunächst an der Luft und dann im Vakuum über Phosphorpentoxid.

Ausbeute: 29.5 g (59 %, Diastereomer 1 / Diasteromer 2 = 79/21)

Nach drei weiteren Kristallisationen aus jeweils 65 ml Dimethylacetamid und 450 ml n-Propanol erhält man 8.0 g (25 %) des Diastereomeren 1 in mehr als 97-%iger Reinheit.
Die spektroskopischen Daten entsprechen denen des Diastereomeren 1 im Ausführungsbeispiel 1. Das Diastereomerenverhältnis wird mittels HPLC bestimmt (LiChrospher 60, RP-select B, 125 x 4 mm, Methanol/Wasser = 5/6 mit 0.12 % Ammoniumdihydrogenphosphat, pH = 2.3; Flußrate: 1 ml/min; Detektion bei $\lambda$ = 228 nm; Retentionszeiten: Diastereomer 1: 14.6 min, Diastereomer 2: 11.7 min).

Ausführungsbeispiel 3:

Stufe 1:

Natrium-3-methoxymethyl-7-phenoxyacetamido-3-cephem-4-carboxylat

Das Natriumsalz wird aus der Carbonsäure (Fujimoto et al., J. Antibiotics XL (1987) 370-84 ) erhalten.
50.3 g (133 mmol) der Carbonsäure und 11.7 g (140 mmol) Natriumhydrogencarbonat werden mit 900 ml Wasser gerührt. Nach dem Filtrieren und Gefriertrocknen erhält man das Natriumsalz.

Ausbeute: 47.8 g (67 %)
$^1$H-NMR ($D_2O$, 270 MHz): $\delta$ = 3.28 (s, 3H, $OCH_3$); 3.42 (AB-System, 2H, $SCH_2$); 4.16 (AB-System, 2H, $CH_2O$); 4.72 (AB-System, 2H, $OCH_2CO$); 5.12 (d, 1H, H-6); 5.67 (d, 1H, H-7); 6.98-7.12 und 7.32-7.42 (2 x m, 5H, Aromaten-H).

Stufe 2:

3-Methoxymethyl-7-phenoxyacetamido-3-cephem-4-carbonsäure-1-(2,2-dimethyl propionyloxy)-ethylester

42.8 g (107 mmol) Natrium-3-methoxymethyl-7-phenoxyacetamido-3-cephem-4-carboxylat in 430 ml trockenem Dimethylformamid werden mit 25.7 g (100 mmol) 2,2-Dimethylpropionsäure-1-iodethylester versetzt. Die Reaktionsmischung wird noch 1 h bei Raumtemperatur gerührt und dann auf eine Mischung aus 2.5 l Wasser und 1.5 l Ethylacetat gegossen. Die wäßrige Phase wird nochmals mit Ethylacetat extrahiert. Die vereinigten organischen Phasen werden mit gesättigter Kochsalzlösung gewaschen, über Magnesiumsulfat getrocknet und im Vakuum eingedampft.

Ausbeute: 50.3 g (98 %, Diastereomer 1 / Diastereomer 2 = 50/50)

Stufe 3:

Chromatographische Diastereomerentrennung

Durch Mitteldruckchromatographie wird die in Stufe 2 erhaltene Diastereomerenmischung getrennt (Kieselgel: 35 - 70 $\mu$m, 1 g Substanz auf 40 g Kieselgel; Toluol/Ethylacetat/Diisopropylether = 120/15/6).

Diastereomer 1:

$^1$H-NMR (DMSO-$d_6$, 270 MHz): $\delta$ = 1.14 (s, 9H, $C(CH_3)_3$); 1.48 (d, 3H, $OCH(CH_3)O$); 3.21 (s, 3H, $OCH_3$); 3.59 (AB-System, 2H, $SCH_2$); 4.14 (s, 2H, $CH_2O$); 4.52 (d, 2H, $OCH_2CO$); 5.18 (d, 1H, H-6); 5.78 (dd, 1H, H-7); 6.87 (q, 1H, $OCH(CH_3)O$); 6.9-7.0 und 7.25-7.32 (2 x m, 5H, Aromaten-H); 9.13 (d, 1H, Amid-NH).

Diastereomer 2:

$^1$H-NMR (DMSO-$d_6$, 270 MHz): $\delta$ = 1.17 (s, 9H, $C(CH_3)_3$); 1.49 (d, 3H, $OCH(CH_3)O$); 3.22 (s, 3H, $OCH_3$); 3.60 (AB-System, 2H, $SCH_2$); 4.13 (s, 2H, $CH_2O$); 4.52 (d, 2H, $OCH_2CO$); 5.18 (d, 1H, H-6); 5.75 (dd, 1H, H-7); 6.90-6.99

(m, 4H, Aromaten-H und $OCH(CH_3)O$); 7.20-7.32 (m, 2H, Aromaten-H); 9.12 (d, 1H, Amid-NH);.

Stufe 4:

7-Amino-3-methoxymethyl-3-cephem-4-carbonsäure-1-(2,2-dimethylpropionyloxy)-ethylester

Diasteromer-1-(p-toluolsulfonat):

Eine Lösung von 3.93 g (7.8 mmol) des Diastereomeren 1 der Stufe 3 und 1.07 ml (8.45 mmol) N,N-Dimethylanilin in 39 ml wasserfreiem Methylenchlorid wird bei - 40 °C tropfenweise mit 1.94 g (9.32 mmol) Phosphorpentachlorid in 32 ml wasserfreiem Methylenchlorid versetzt, wobei die Innentemperatur - 25 °C nicht übersteigen sollte. Innerhalb von 2 h läßt man die Temperatur auf - 10 °C ansteigen und setzt dann in einer Portion 19.4 ml iso-Butanol zu. Nach 10 min gießt man die Reaktionslösung auf 250 ml gesättigte Natriumhydrogencarbonatlösung und 250 ml Ethylacetat und trennt die organische Phase möglichst schnell ab. Die wäßrige Phase wird nochmals mit Ethylacetat extrahiert. Dann werden die vereinigten organischen Phasen mit gesättigter Kochsalzlösung gewaschen, über Magnesiumsulfat getrocknet und im Vakuum zur Trockne eingeengt. Der Rückstand wird in 5 ml Ethylacetat gelöst und mit einer Lösung von 1.47 g (7.74 mmol) p-Toluolsulfonsäure-monohydrat in 10 ml Ethylacetat versetzt. Das Produkt wird abgesaugt, mit Ethylacetat gewaschen und im Vakuum über Phosphorpentoxid getrocknet.

Ausbeute 2.57 g (61 %)
$^1H$-NMR (DMSO-$d_6$, 270 MHz): $\delta$ = 1.15 (s, 9H, $C(CH_3)_3$); 1.48 (d, 3H, $OCH(CH_3)O$); 2.29 (s, 3H, Aryl-$CH_3$); 3.23 (s, 3H, $OCH_3$); 3.69 (AB-System, 2H, $SCH_2$); 4.16 (s, 2H, $CH_2O$); 5.24 und 5.28 (2 x d, 2 x 1H, H-6 und H-7); 6.87 (q, 1H, $OCH(CH_3)O$); 7.12 (d, 2H, Aromaten-H); 7.49 (d, 2H, Aromaten-H); 8.88 (s, 2H, $NH_2$).

Diastereomer-2-hydrochlorid:

Ausgehend von 506 mg (1 mmol) Diastereomer 2 der Stufe 2 führte man analog die Abspaltung der Phenoxyacetylgruppe durch. Diastereomer 2 kristallisiert als Hydrochlorid aus Ethylacetat.

Ausbeute: 223 mg (55 %)
$^1H$-NMR (DMSO-$d_6$, 270 MHz): $\delta$ = 1.17 (s, 9H, $C(CH_3)_3$); 1.49 (d, 3H, $OCH(CH_3)O$); 3.24 (s, 3H, $OCH_3$); 3.68 (AB-System, 2H, $SCH_2$); 4.20 (s, 2H, $CH_2O$); 5.21 und 5.25 (2 x d, 2 x 1H, H-6 und H-7); 6.93 (q, 1H, $OCH(CH_3)O$); 9.18 (s, 2H, $NH_2$).

Stufe 5:

3-Methoxymethyl-7-[2-(2-tritylaminothiazol-4-yl)-2-(Z)-trityloximinoacetamido-3-cephem-4-carbonsäure-1-(2,2-dimethylpropionyloxy)-ethylester(Diastereomer 1)

1.5 g (2.75 mmol) Diastereomer-1-tosylat aus Stufe 4 wird in 100 ml Ethylacetat und 30 ml Wasser suspendiert. Unter kräftigem Rühren stellt man bei 0 °C mit gesättigter Natriumhydrogencarbonatlösung den pH-Wert auf 6.5 ein. Die organische Phase wird nacheinander mit je 30 ml Wasser und gesättigter Kochsalzlösung gewaschen, über Natriumsulfat getrocknet und im Vakuum zur Trockne eingeengt.

Ausbeute: 1.03 g (98 %)

Wie im Ausführungsbeispiel 1 beschrieben, überführt man 1.81 g (2.3 mmol) 2-(2-Tritylaminothiazol-4-yl)-2-(Z)-trityloximinoessigsäure-triethylammoniumsalz in das Säurechlorid.

Zu einer Lösung von 880 mg (2.3 mmol) Ester in 10 ml wasserfreiem Methylenchlorid tropft man bei - 5 °C das Säurechlorid in 8 ml wasserfreiem Methylenchlorid zu. Nach 2 h wird aufgearbeitet, wie im Ausführungsbeispiel 1 beschrieben wurde. Nach der Chromatographie des Rohproduktes mit Toluol/Ethylacetat (5/1) erhält man diastereomerenreines Produkt.

Ausbeute: 2.38 g (99 %)

Die spektroskopischen Daten entsprechen denen von Diastereomer 1 im Ausführungsbeispiel 1. Die weiteren Umsetzungen werden wie dort beschrieben durchgeführt.

Ausführungsbeispiel 4:

7-Amino-3-methoxymethyl-3-cephem-4-carbonsäure-1-(2,2-dimethylpropionyloxy)-ethylester, Diastereomer-1-tosylat und Diastereomer-2-hydrochlorid

Wie für die Stufe 4 im Ausführungsbeispiel 3 beschrieben, führt man die Abspaltung der Phenoxyacetylgruppe ausgehend von 3.03 g (6 mmol) 3-Methoxymethyl-7-Phenoxyacetamido-3-cephem-4-carbonsäure-1-(2,2-dimethyl propionyloxy)-ethylester (Diastereomer 1 / Diastereomer 2 = 52/48) durch. Die getrockneten organischen Phasen werden auf 10 ml eingeengt. Beim Abkühlen auf 0 °C fällt das Diastereomere 2 als Hydrochlorid aus und wird abgesaugt (Ausbeute: 759 mg = 31 %). Die Mutterlauge wird mit 1.13 g (5.9 mmol) p-Toluolsulfonsäure-monohydrat in 5 ml Ethylacetat versetzt. Das ausgefallene Toluolsulfonsäuresalz wird abgesaugt, mit wenig Ethylacetat gewaschen und im Vakuum über Phosphorpentoxid getrocknet.

Ausbeute: 667 mg (23 %)

Der Gehalt an Diastereomerem 1 liegt nach HPLC über 97 Prozent (HPLC: LiChrospher 100 RP-18, 5 µm, 125 x 4 mm, Flußrate: 1 ml/min, Detektion bei $\lambda$ = 254 nm, Wasser/Methanol = 52/48 mit 0.1 % Ammoniumacetat, Retentionszeiten: Diastereomer 1: 12.1 min, Diastereomer 2: 11.8 min).

Die Überführung der Zwischenstufe in das Endprodukt wurde bereits in den vorhergehenden Ausführungsbeispielen beschrieben.

Ausführungsbeispiel 5:

7-Amino-3-methoxymethyl-3-cephem-4-carbonsäure-1-(2,2-dimethylpropionyloxy)-ethylester, Diastereomer-1-p-toluolsulfonat

Eine Suspension von 4.88 g (20 mmol) 7-Amino-3-methoxymethyl-3-cephem-4-carbonsäure in 200 ml wasserfreiem Methylenchlorid wird bei 0 °C mit 3.12 ml (21 mmol) DBU versetzt. Zu der leicht getrübten, gelben Lösung setzt man 4.99 g (24 mmol) 2,2-Dimethylpropionsäure-1-bromethylester zu und rührt dann 3 h bei Raumtemperatur. Die Reaktionslösung wird auf 600 ml gesättigte Natriumhydrogencarbonatlösung und 800 ml Methylenchlorid gegossen. Die organische Phase wird mit gesättigter Kochsalzlösung gewaschen, über Natriumsulfat getrocknet und im Vakuum eingedampft. Das Rohprodukt (9.3 g) wird in 15 ml Ethylacetat gelöst und mit 1.9 g (10 mmol) p-Toluolsulfonsäure-monohydrat in 10 ml Ethylacetat versetzt. Das ausgefallene Produkt wird abgesaugt, mit Diisopropylether gewaschen und im Vakuum getrocknet.

Ausbeute: 3.95 g (36 %) Diastereomer 1 / Diastereomer 2 = 85/15

Die Umkristallisation des Salzes aus n-Propanol ergibt reines Diastereomeres 1, das wie beschrieben weiter umgesetzt wird.

Ausführungsbeispiel 6:

7-Amino-3-methoxymethyl-3-cephem-4-carbonsäure-1-(2,2-dimethylpropionyloxy)-ethylester, Diastereomer-1-tosylat und Diastereomer-2-hydrochlorid

Wie im Ausführungsbeispiel 5 beschrieben, stellt man aus 4.88 g (20 mmol) 7-Amino-3-methoxymethyl-3-cephem-4-carbonsäure das Rohprodukt her. Das so erhaltene Öl wird in 20 ml Ethylacetat gelöst und mit einer frisch bereiteten Lösung von 0.65 ml (9.2 mmol) Acetylchlorid und 1.07 ml (18.4 mmol) Ethanol in 5 ml Ethylacetat versetzt. Das im Eisbad ausfallende Hydrochlorid wird abgesaugt, mit Ethylacetat gewaschen und getrocknet.

Ausbeute: 2.57 g (32 %), Diastereomer 1 / Diastereomer 2 = 22/78

Das Filtrat wird mit einer Lösung von 1.75 g (9.2 mmol) p-Toluolsulfonsäure-monohydrat in 8 ml Ethylacetat versetzt und der ausgefallene Niederschlag abgesaugt.

Ausbeute: 1.15 g (16 %), Diastereomer 1 /Diastereomer 2 = 97/3

Die spektroskopischen Daten entsprechen denen im Ausführungsbeispiel 3.

Ausführungsbeispiel 7:

Stufe 1:

3-Methoxymethyl-7-[(napht-2-yl)-methylidenamino]-3-cephem-4-carbonäure-1-(2,2-dimethylpropionyloxy)-ethylester (Diastereomerengemisch)

Wie im Ausführungsbeispiel 5 beschrieben, stellt man aus 2.44 g (10 mmol) 7-Amino-3-methoxymethyl-3-cephem-4-carbonsäure den rohen Ester her. Das so erhaltene Öl wird in 30 ml wasserfreiem Methylenchlorid gelöst und mit einer Lösung von 1.56 g (10 mmol) Naphthalin-2-carbaldehyd in 40 ml Toluol versetzt. Nach 3 h bei Raumtemperatur wird mit 40 ml Toluol verdünnt und dreimal mit je 30 ml Wasser gewaschen. Die Lösung wird über Magnesiumsulfat getrocknet und im Vakuum zur Trockne eingeengt.

Stufe 2:

Chromatographische Trennung der Diastereomeren

Das Rohprodukt aus Stufe 1 wird an 500 g Kieselgel (pH 7.5) chromatographiert. Dazu wird käufliches Kieselgel (35 - 70 $\mu$m) in Wasser suspendiert und mit verdünnter Natronlauge versetzt, bis der pH-Wert von 7.5 konstant bleibt. Das Kieselgel wird abgesaugt, mit Methanol gewaschen und 18 h bei 110 °C/10,027 bar (20 Torr) getrocknet. Mit Toluol/Ethylacetat (20/1) eluiert man zunächst 1.7 g (33 %) Diastereomer 1 und dann 1.65 g (32 %) Diastereomer 2. Diastereomer 1 kristallisiert aus Methanol in farblosen Nadeln vom Schmelzpunkt 110 °C.

Diastereomer 1:

$^1$H-NMR ($CDCl_3$, 270 MHz): $\delta$ = 1.22 (s, 9H, $C(CH_3)_3$); 1.58 (d, 3H, $CH-CH_3$); 3.22 (s, 3H, $OCH_3$); 3.57 (s, 2H, $SCH_2$); 4.31 (AB-System, 2H, $CH_2O$); 5.21 (d, 1H, H-6); 5.50 (dd, 1H, H-7); 6.99 (q, 1H, $CH-CH_3$); 7.52 (mc, 2H, Aromaten-H), 7.88 (mc, 3H, Aromaten-H); 8.03 (mc, 3H, Aromaten-H); 8.78 (d, 1H, CH=N).

Diastereomer 2:

$^1$H-NMR ($CDCl_3$, 270 MHz): $\delta$ = 1.22 (s, 9H, $C(CH_3)_3$); 1.58 (d, 3H, $CH-CH_3$); 3.32 (s, 3H, $OCH_3$); 3.52 (s, 2H, $SCH_2$); 4.26 (AB-System, 2H, $CH_2O$); 5.26 (d, 1H, H-6); 5.49 (dd, 1H, H-7); 7.02 (q, 1H, $CH-CH_3$); 7.51 (mc, 2H, Aromaten-H), 7.84 (mc, 3H, Aromaten-H); 8.02 (mc, 3H, Aromaten-H); 8.75 (d, 1H, CH = N).

Die Spaltung der Schiffschen Basen zu den reinen Diastereomeren von 7-Amino-3-methoxymethyl-3-cephem-4-carbonsäure-1-(2,2-dimethyl-propionyloxy)-ethylester erfolgt mit Girard T-Reagens analog zu Literaturvorschriften (z. B. Kamachi et al., The Journal of Antibiotics XLI (11) (1988), 1602-1616).

## Patentansprüche

**Patentansprüche für folgende Vertragsstaaten : AT, BE, CH, DE, DK, FR, GB, IT, LI, LU, NL, PT, SE**

1. Kristallines Säureadditionssalz des 3-Cephem-4-carbonsäure-1-(2,2-dimethylpropionyloxy)-ethylesters der allgemeinen Formel II

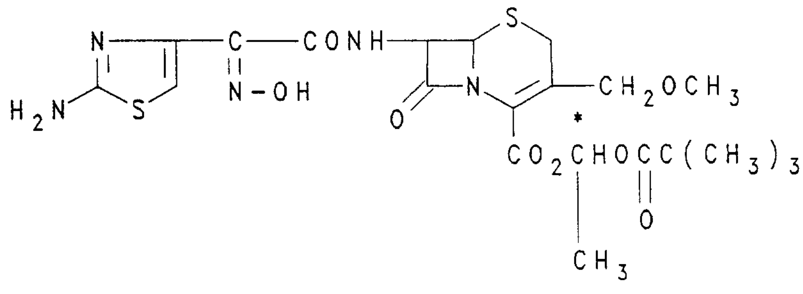

x HX II

worin das mit * gekennzeichnete C-Atom (1S)-Konfiguration aufweist, die Gruppe = N-OH in syn-Position steht und

X für das Anion einer physiologisch unbedenklichen, ein- oder mehrbasischen, anorganischen oder organischen Säure steht.

2. Kristallines Säureadditionssalz des 3-Cephem-4-carbonsäure-1-(2,2-dimethylpropionyloxy)-ethylesters der allgemeinen Formel II gemäß Anspruch 1, worin HX für Benzolsulfonsäure, p-Toluolsulfonsäure oder 4-Ethylbenzolsulfonsäure steht.

3. Verfahren zur Herstellung von Verbindungen der allgemeinen Formel II gemäß den Ansprüchen 1 oder 2, dadurch gekennzeichnet, daß

a) eine Verbindung der Formel III hergestellt wird,

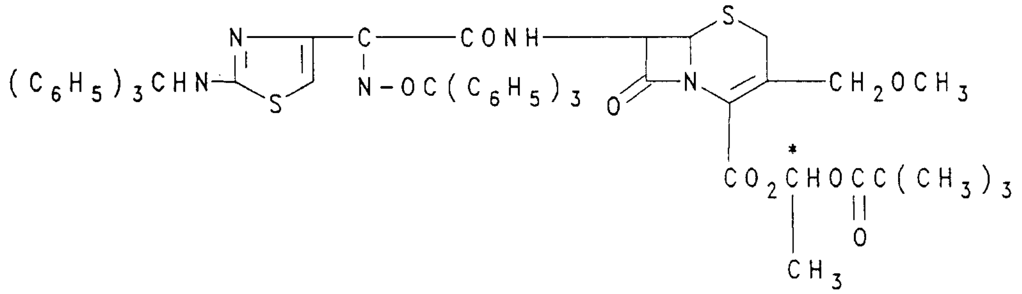

III

die Diastereomeren durch Chromatographie getrennt, die Tritylgruppen abgespalten, die Säureadditionsprodukte hergestellt werden oder

b) aus der Mischung der Diastereomeren der Formel II durch Kristallisation das weniger polare Diastereomere angereichert wird oder

c) eine Zwischenstufe der Formel IV in Form der getrennten Diastereomeren hergestellt wird

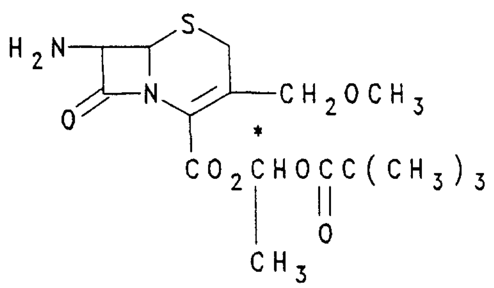

IV

und in die getrennten Diastereomeren der Formel II übergeführt wird.

4. Gegen bakterielle Infektionen wirksame pharmazeutische Zubereitungen gekennzeichnet durch einen Gehalt an kristallinen Säureadditionssalzen des 3-Cephem-4-carbonsäure-1-(2,2-dimethylpropionyl-oxy)-ethylesters der allgemeinen Formel II gemäß den Ansprüchen 1 oder 2.

5. Verfahren zur Herstellung von gegen bakteriellen Infektionen wirksamen pharmazeutischen Zubereitungen gemäß Anspruch 4, dadurch gekennzeichnet, daß ein kristallines Säureadditionssalz des 3-Cephem-4-carbonsäure-1-(2,2-dimethylpropionyloxy)-ethylesters der allgemeinen Formel II mit pharmazeutisch üblichen Trägerstoffen oder Verdünnungsmitteln in eine pharmazeutisch geeignete Verabreichungsform gebracht wird.

6. Verwendung von kristallinen Säureadditionssalzen des 3-Cephem-4-carbonsäure-1-(2,2-dimethylpropionyloxy)-ethylesters der allgemeinen Formel II gemäß den Ansprüchen 1 oder 2 zur Herstellung eines Arzneimittels zur Bekämpfung bakterieller Infektionen.

**Patentansprüche für folgende Vertragsstaaten : ES, GR**

1. Verfahren zur Herstellung von kristallinen Säureadditionssalze des 3-Cephem-4-carbonsäure-1-(2,2-dimethylpropionyloxy)-ethylesters der allgemeinen Formel II

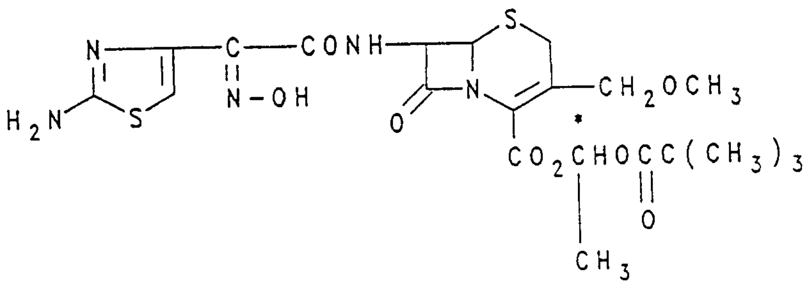

x HX    II

worin das mit * gekennzeichnete C-Atom (1S)-Konfiguration aufweist, die Gruppe = N-OH in syn-Position steht und X für das Anion einer physiologisch unbedenklichen, ein- oder mehrbasischen, anorganischen oder organischen Säure steht, dadurch gekennzeichnet, daß

a) eine Verbindung der Formel III hergestellt wird,

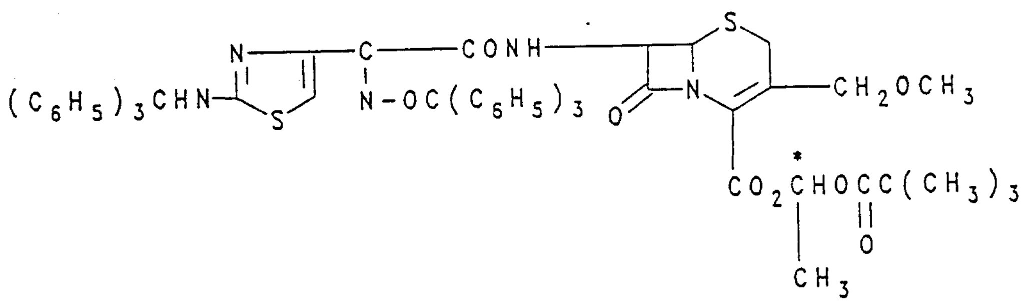

III

die Diastereomeren durch Chromatographie getrennt, die Tritylgruppen abgespalten, die Säureadditionsprodukte hergestellt werden oder

b) aus der Mischung der Diastereomeren der Formel II durch Kristallisation das weniger polare Diastereomere angereichert wird oder

c) eine Zwischenstufe der Formel IV in Form der getrennten Diastereomeren hergestellt wird

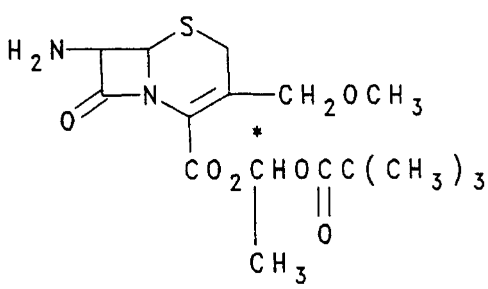

IV

und in die getrennten Diastereomeren der Formel II übergeführt wird.

2. Verfahren zur Herstellung kristalliner Säureadditionssalze des 3-Cephem-4-carbonsäure-1-(2,2-dimethylpropionyloxy)-ethylesters der allgemeinen Formel II gemäß Anspruch 1, worin HX für Benzolsulfonsäure, p-Toluolsulfonsäure oder 4-Ethylbenzolsulfonsäure steht.

3. Verfahren zur Herstellung von gegen bakterielle Infektionen wirksamen pharmazeutischen Zubereitungen mit einem Gehalt an kristallinen Säureadditionssalzen des 3-Cephem-4-carbonsäure-1-(2,2-dimethylpropionyloxy)-ethylesters der allgemeinen Formel II gemäß den Ansprüchen 1 oder 2, worin ein kristallines Säureadditionssalz des 3-Cephem-4-carbonsäure-1-(2,2-dimethylpropionyloxy)-ethylesters der allgemeinen Formel II mit pharmazeutisch üblichen Trägerstoffen oder Verdünnungsmitteln in eine pharmazeutisch geeignete Verabreichungsform gebracht wird.

## Claims

### Claims for the following Contracting States : AT, BE, CH, DE, DK, FR, GB, IT, LI, LU, NL, PT, SE

1. A crystalline acid addition salt of the 1-(2,2-dimethylpropionyloxy)ethyl 3-cephem-4-carboxylate of the formula II

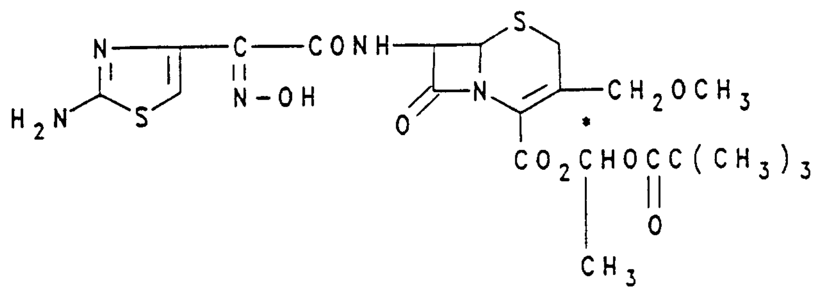

x HX

II

in which the C atom labeled with * has the (1S)-configuration, the group =N-OH is in the syn-position and X is the anion of a physiologically acceptable, mono- or polybasic, inorganic or organic acid.

2. A crystalline acid addition salt of the 1-(2,2-dimethylpropionyloxy) ethyl 3-cephem-4-carboxylate of the formula II as claimed in claim 1, in which HX is benzenesulfonic acid, p-toluenesulfonic acid or 4-ethylbenzenesulfonic acid.

3. A process for the preparation of a compound of the formula II as claimed in claims 1 or 2, which comprises

a) preparing a compound of the formula III

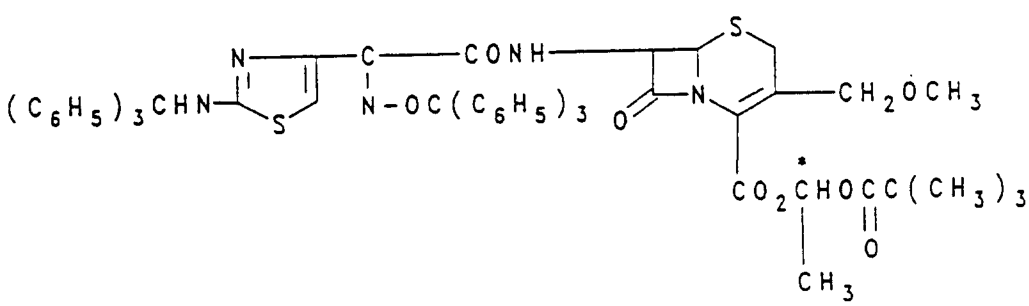

III

separating the diastereomers by chromatography, splitting off the trityl groups and preparing the acid addition products, or

b) concentrating the less polar diastereomer from the mixture of diastereomers of the formula II by crystallization, or

c) preparing an intermediate stage of the formula IV in the form of the separated diastereomers

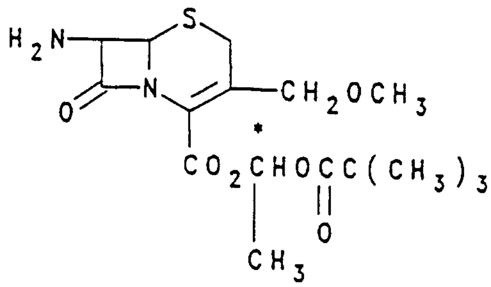

IV

and converting this stage into the separated diastereomers of the formula II.

4. A pharmaceutical formulation which is active against bacterial infections, which has a content of a crystalline acid addition salt of the 1-(2,2-dimethylpropionyloxy)ethyl 3-cephem-4-carboxylate of the formula II as claimed in claims 1 or 2.

5. A process for the preparation of a pharmaceutical formulation which is active against bacterial infections as claimed in claim 4, which comprises bringing a crystalline acid addition salt of the 1-(2,2-dimethylpropionyloxy)ethyl 3-cephem-4-carboxylate of the formula II into a pharmaceutically suitable administration form with pharmaceutically customary excipients or diluents.

6. The use of a crystalline acid addition salt of the 1-(2,2-dimethylpropionyloxy)ethyl 3-cephem-4-carboxylate of the formula II as claimed in claims 1 or 2 for the preparation of a drug for combating bacterial infections.

**Claims for the following Contracting States : ES, GR**

1. A process for the preparation of a crystalline acid addition salt of the 1-(2,2-dimethylpropionyloxy)ethyl 3-cephem-4-carboxylate of the formula II

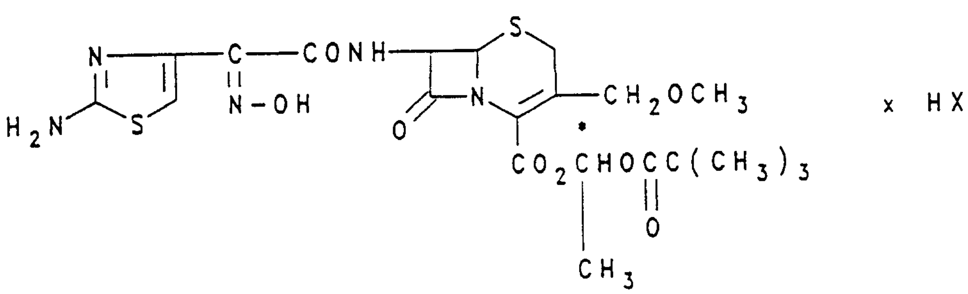

II

in which the C atom labeled with * has the (1S)-configuration, the group =N-OH is in the syn-position and X is the anion of a physiologically acceptable, mono- or polybasic, inorganic or organic acid, which comprises

a) preparing a compound of the formula III

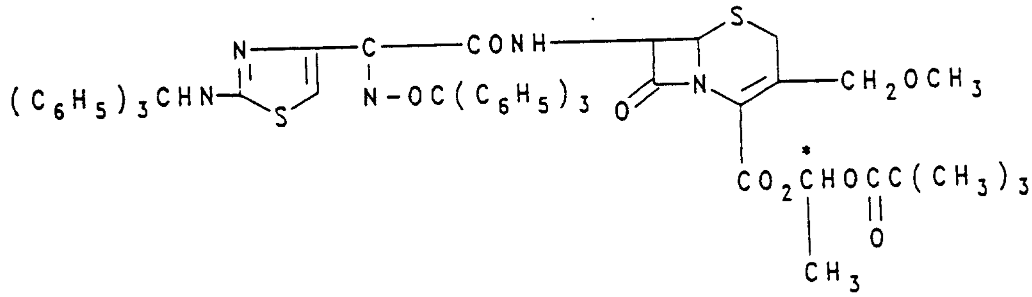

III

separating the diastereomers by chromatography, splitting off the trityl groups and preparing the acid addition products, or

b) concentrating the less polar diastereomer from the mixture of diastereomers of the formula II by crystallization, or

c) preparing an intermediate stage of the formula IV in the form of the separated diastereomers

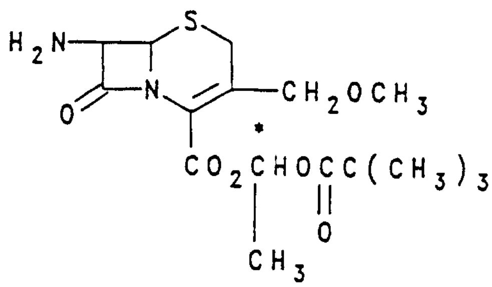

IV

and converting this stage into the separated diastereomers of the formula II.

2. A process for the preparation of a crystalline acid addition salt of the 1-(2,2-dimethylpropionyloxy)ethyl 3-cephem-4-carboxylate of the formula II as claimed in claim 1, in which HX is benzenesulfonic acid, p-toluenesulfonic acid or 4-ethylbenzenesulfonic acid.

3. A process for the preparation of a pharmaceutical formulation which is active against bacterial infections and contains a crystalline acid addition salt of the 1-(2,2-dimethylpropionyloxy)ethyl 3-cephem-4-carboxylate of the formula II as claimed in claims 1 or 2, in which a crystalline acid addition salt of the 1-(2,2-dimethylpropionyloxy)ethyl 3-cephem-4-carboxylate of the formula II is brought into a pharmaceutically suitable administration form with pharmaceutically customary excipients or diluents.

**Revendications**

**Revendications pour les Etats contractants suivants : AT, BE, CH, DE, DK, FR, GB, IT, LI, LU, NL, PT, SE**

1. Sel cristallin d'addition avec un acide du 3-céphème-4-carboxylate de 1-(2,2-diméthylpropionyloxy)éthyle de formule générale II

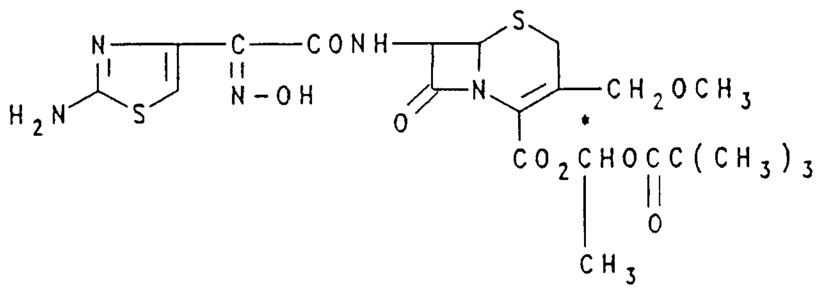 x HX II

dans laquelle l'atome de carbone désigné par * présente la configuration (1S), le groupe =N-OH est en position *syn* et X représente l'anion d'un mono- ou polyacide organique ou minéral, physiologiquement acceptable.

**2.** Sel cristallin d'addition avec un acide du 3-céphème-4-carboxylate de 1-(2,2-diméthylpropionyloxy)éthyle de formule générale II selon la revendication 1, dans lequel HX représente l'acide benzènesulfonique, l'acide p-toluènesulfonique ou l'acide 4-éthylbenzènesulfonique.

**3.** Procédé pour la préparation des composés de formule générale II selon la revendication 1 ou 2, caractérisé en ce que

a) on prépare un composé de formule III

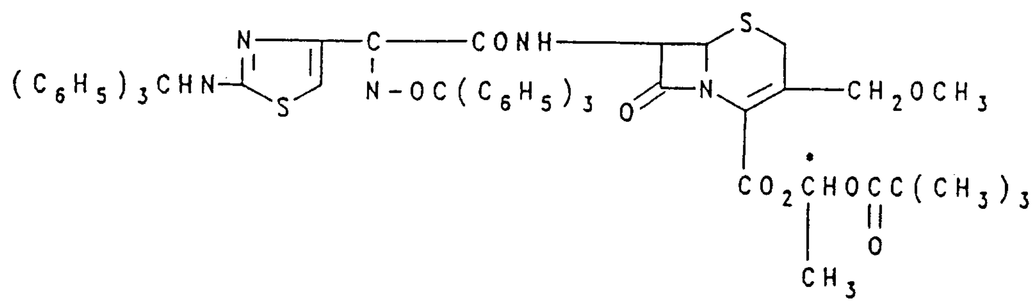 III

on sépare les diastéréoisomères par chromatographie, on élimine les groupes trityle, on prépare les produits d'addition avec des acides, ou

b) à partir du mélange des diastéréoisomères de formule II, on concentre par cristallisation le diastéréoisomère le moins polaire, ou

c) on prépare un composé intermédiaire de formule IV, sous forme des diastéréoisomères séparés

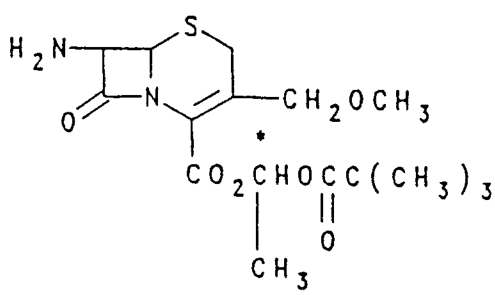 IV

et on le convertit en les diastéréoisomères de formule II séparés.

**4.** Compositions pharmaceutiques actives contre des infections bactériennes, caractérisées par une teneur en sels cristallins d'addition avec des acides du 3-céphème-4-carboxylate de 1-(2,2-diméthylpropionyloxy)éthyle de formule générale II, selon la revendication 1 ou 2.

5. Procédé pour la préparation de compositions pharmaceutiques actives contre des infections bactériennes selon la revendication 4, caractérisé en ce que l'on met sous une forme d'administration pharmaceutiquement appropriée un sel cristallin d'addition avec un acide du 3-céphème-4-carboxylate de 1-(2,2-diméthylpropionyloxy)éthyle de formule générale II, avec des véhicules ou diluants pharmaceutiquement usuels.

6. Utilisation de sels cristallins d'addition avec des acides du 3-céphème-4-carboxylate de 1-(2,2-diméthylpropionyloxy)éthyle de formule générale II selon la revendication 1 ou 2, pour la fabrication d'un médicament destiné à la lutte contre des infections bactériennes.

**Revendications pour les Etats contractants suivants : ES, GR**

1. Procédé pour la préparation de sels cristallins d'addition avec un acide du 3-céphème-4-carboxylate de 1-(2,2-diméthylpropionyloxy)éthyle de formule générale II

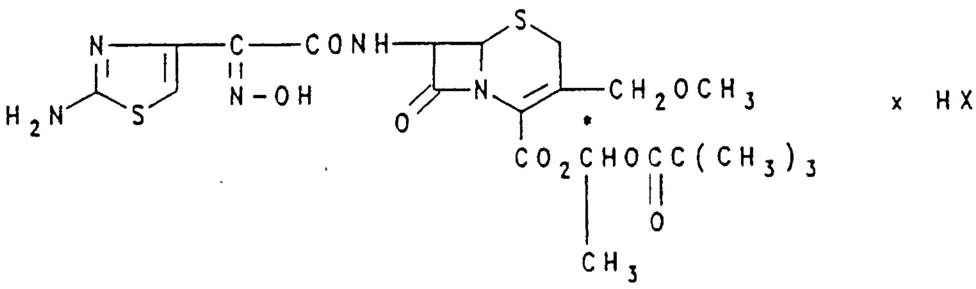

II

dans laquelle l'atome de carbone désigné par * présente la configuration (1S), le groupe =N-OH est en position *syn* et X représente l'anion d'un mono- ou polyacide organique ou minéral, physiologiquement acceptable, caractérisé en ce que

a) on prépare un composé de formule III

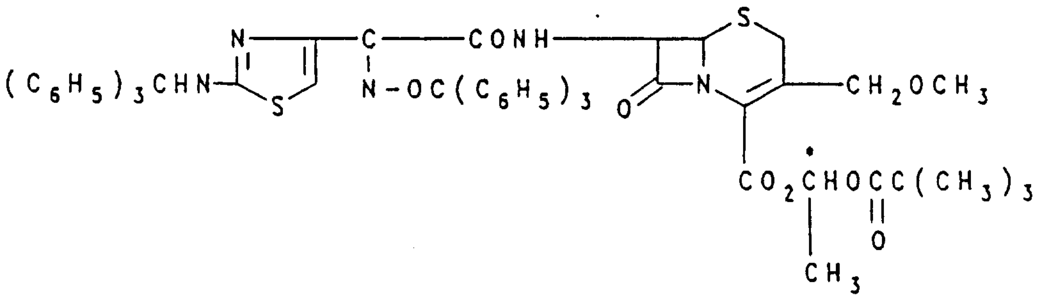

III

on sépare les diastéréoisomères par chromatographie, on élimine les groupes trityle, on prépare les produits d'addition avec des acides, ou

b) à partir du mélange des diastéréoisomères de formule II, on concentre par cristallisation le diastéréoisomère le moins polaire, ou

c) on prépare un composé intermédiaire de formule IV, sous forme des diastéréoisomères séparés

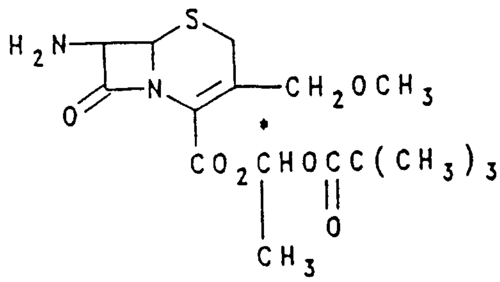

IV

et on le convertit en les diastéréoisomères de formule II séparés.

2. Procédé pour la préparation de sels cristallins d'addition avec des acides du 3-céphème-4-carboxylate de 1-(2,2-diméthylpropionyloxy)éthyle de formule générale II selon la revendication 1, dans lesquels HX représente l'acide benzènesulfonique, l'acide p-toluènesulfonique ou l'acide 4-éthylbenzènesulfonique.

3. Procédé pour la préparation de compositions pharmaceutiques actives contre des infections bactériennes, ayant une teneur en sels cristallins d'addition avec des acides du 3-céphème-4-carboxylate de 1-(2,2-diméthylpropionyloxy)éthyle de formule générale II, selon la revendication 1 ou 2, dans lequel on met sous une forme d'administration pharmaceutiquement appropriée un sel cristallin d'addition avec un acide du 3-céphème-4-carboxylate de 1-(2,2-diméthylpropionyloxy)éthyle de formule générale II, avec des véhicules ou diluants pharmaceutiquement usuels.